# EUROPEAN PATENT APPLICATION

(11) **EP 2 801 385 A1**
(43) Date of publication of application: **12.11.2014**
(21) Application number: 13166836.0
(22) Date of filing: 07.05.2013
(51) Int. Cl.: A61M 25/01, A61M 25/10, A61M 25/00

(54) **Balloon catheter**

(71) Applicant: IMDS R&D BV, 9301 NZ Roden (NL)
(72) Inventor: Schulting, Edwin Alexander, 9301 NZ Roden (NL); Boudewijn, Alexander Christiaan, 9301 NZ Roden (NL); Rijpkema, Jacob, 9301 NZ Roden (NL)
(74) Representative: Jansen, Cornelis Marinus

(57) **Abstract**

A balloon catheter has a stiffening member (16) extending in an inflation lumen (6) from a proximal end of a shaft body (5) and having a distal end portion (17) in a balloon carrier (11) connected to and projecting distally from a distal end portion (17) of the shaft body (5). A guidewire lumen (19) extends from a proximal guidewire port (20) at or near a proximal end of the balloon (12), through the balloon carrier (11) and the balloon (12), to a distal guidewire port (21) at or near a distal end of the balloon (12). A wall between the guidewire lumen (19) and the inflation lumen (6) has a portion oriented obliquely away from the proximal guidewire port (20) in a section of the catheter between proximal and distal ends of the proximal guidewire port (20). The distal end portion (17) of the stiffening member (16) is fixed to the obliquely oriented wall portion.

## Description

### FIELD AND BACKGROUND OF THE INVENTION

The invention relates to a balloon catheter and more specifically to a rapid exchange balloon catheter.

From US 6 605 057 a rapid exchange balloon catheter is known with a shaft section having a shaft body bounding a shaft portion of an inflation lumen and a balloon section distally of the shaft section. A balloon is sealingly coupled to a balloon carrier of the balloon section. The balloon is inflatable from a slender configuration extending tightly around the balloon carrier to a radially inflated configuration by supplying air via the inflation lumen into an internal space of the balloon.

A stiffening member in the form of a wire extends through the shaft body from the proximal end of the shaft section to the distal end of the shaft section and a guidewire lumen extends from a proximal guidewire port in a circumferential surface of the balloon carrier near a proximal end of the balloon, through the balloon carrier and the balloon, to a distal guidewire port near a distal end of the balloon.

As is typical for a rapid exchange catheter, a guide wire port is provided in a distal portion of the catheter for facilitating placement of a catheter over a guide wire arranged in a patient and removal of the catheter from a guide wire while leaving the distal guide wire end positioned inside the patent. The guide wire port is located in an area where an exchange joint sleeve is fused to the catheter shaft for additional strength. To further stiffen the catheter in this area, the guide wire tube is fused with the catheter shaft to form a fused polyethylene laminate. Mandrels are temporarily positioned between the guide wire tube and the catheter shaft and inside the guide wire tube for keeping respective passages open and a heat source is applied where the fused laminate is desired. After the fused laminate is formed, the mandrels are withdrawn.

The stiffening wire is attached at spaced locations along its length to the inner wall of the catheter shaft, for instance by wire "tacks" or sleeves that are attached to the stiffening wire before the stiffening wire is inserted into the catheter. After the stiffening wire has been placed within the catheter shaft, the wire tacks, sleeves are attached to the inner wall of the catheter shaft.

The stiffening wire has a large diameter proximal portion and a smaller diameter or tapered distal portion for increased flexibility in the region of the exchange joint.

EP 1 051 990 A1 discloses a rapid exchange catheter which has a tapered stiffening wire of which a section with narrowed cross-section extends along a guidewire port and towards a balloon section composed of tubes to which a balloon is sealed.

US 2006/0270977 discloses a rapid exchange catheter having two stiffening members. The first stiffening member constituted by e.g. a hypotube with a tapered end in an inflation lumen of the catheter. The second stiffening member includes a core wire embedded in the catheter shaft and extends generally in parallel to the inflation lumen. The flexibility of the second stiffening member decreases distally. The second stiffening member includes a coil positioned within the catheter shaft proximal of the balloon and of the proximal port of the guidewire lumen and tapering with the core wire for adding resiliency to the catheter shaft and enhance the flexibility of the shaft proximal of the balloon and the guidewire port. A guidewire lumen shaft ends at its proximal end adjacent to the guidewire port and forms the guidewire lumen.

A problem of such catheters is that manufacturing is quite complicated and quality control of the various steps is challenging, so the catheters are quite costly. This is of particular relevance, because percutaneous coronary interventions such as percutaneous transluminal coronary angioplasty are carried out in large numbers and constitute interventions requiring relatively little patient care, so the costs of devices used forms a relatively large proportion of overall treatment costs.

### SUMMARY OF THE INVENTION

It is an object of the present invention to provide a balloon catheter with good flexibility for resilience and easy maneuvering through tortuous vessels and with good pushability for passing into or through stenotic sections or occlusions, yet of a more simple construction so that manufacturing costs are reduced and quality assurance is facilitated.

According to the invention, this object is achieved by providing a balloon catheter according to claim 1.

Because the distal end portion of the stiffening member extends is fixed to the wall portion oriented obliquely away from the proximal guidewire port, the distal end portion can be fixed in a simple manner and the stiffening effect of the stiffening member is effectively transferred from the stiffening member to the guidewire extending in the guidewire lumen bounded by that wall portion and generally in alignment with the stiffening member. Pushing forces exerted by the stiffening member are effectively transferred via the oblique wall portion that is generally located in front of the stiffening member axis to the balloon carrier and, accordingly, to the distal side of the balloon. Because the stiffening member extends in the inflation lumen and ends where the guidewire enters, the catheter can be of a very slender design, also at and adjacent the guidewire entry port.

Particular elaborations and embodiments of the invention are set forth in the dependent claims.

Further features, effects and details of the invention appear from the detailed description and the drawings.

### BRIEF DESCRIPTION OF THE DRAWINGS

Fig. 1 is a schematic side view of a catheter according to the invention with some sections with constant cross-section left out;
Fig. 2 is an enlarged view of a portion II in Fig. 1;
Fig. 3 is an enlarged, partially cut-away view of a portion III in Fig. 1;
Fig. 4 is an enlarged view in cross-section along a center line of a portion IV in Fig. 1; and

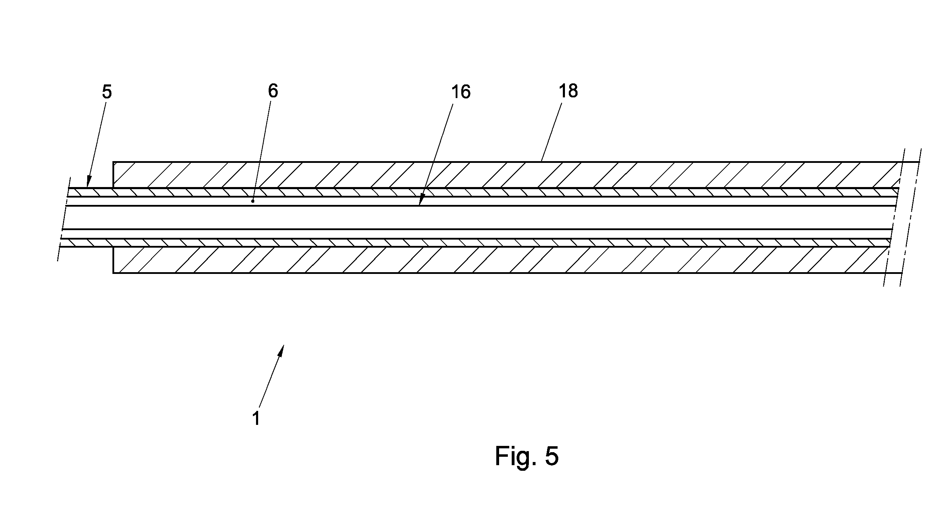

Fig. 5 is an enlarged view in cross-section along a center line of a portion V in Fig. 1.

### DETAILED DESCRIPTION

In the drawings, an example of a balloon catheter 1 according to the invention is shown. The balloon catheter 1 has a shaft section 2 with a shaft body 5 bounding a shaft portion 13 of an inflation lumen 6 extending from a proximal end 7 of the shaft section 2 to a distal end 27. The shaft section 2 projects from a handle section 4 including a handle 9 with a bellows 10 of which an internal space communicates with the inflation lumen 6 and into which the shaft body 5 is inserted and fixed.

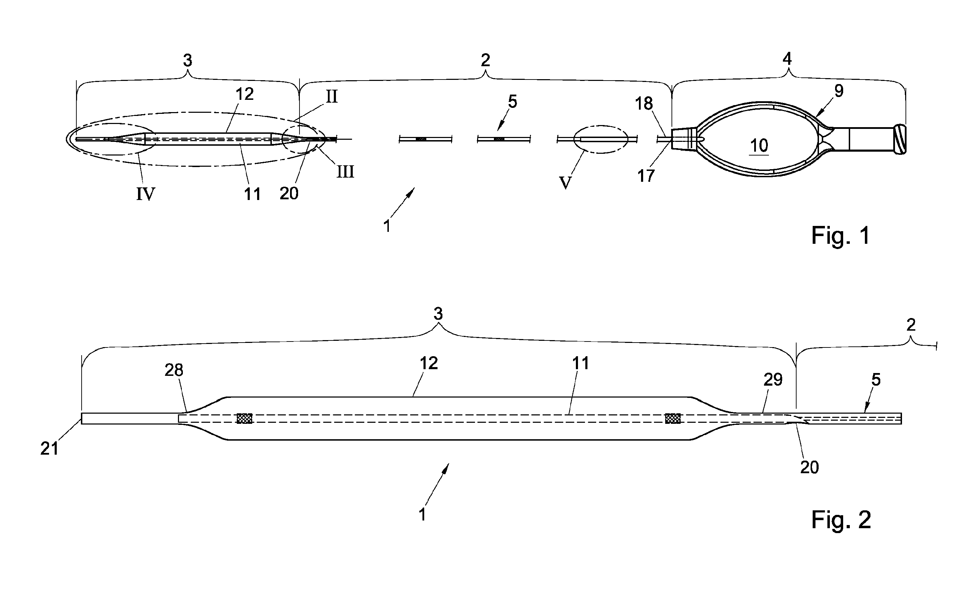

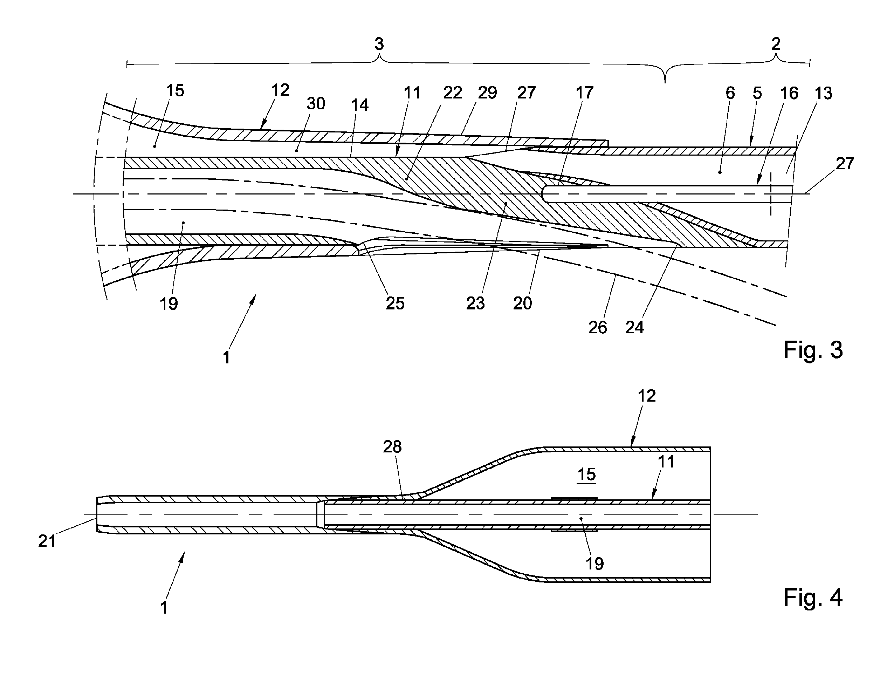

A balloon section 3 distally of the shaft section 2 has a balloon carrier 11 and a balloon 12 sealingly coupled, for instance by an adhesive, to the balloon carrier 11. The balloon carrier 11 is fixed to a distal end portion of the shaft body 5. The inflation lumen 6 communicates with an internal space 15 of the balloon 12. At a distal end 27, the inflation lumen 6 connects to a proximal portion 30 of the internal space 15 of the balloon. The balloon 12 is inflatable from a slender configuration extending tightly around the balloon carrier 11 to a radially inflated configuration as shown in Figs. 1-4 for expanding a stenosis and/or expanding a stent or other expandable device to be deployed at the location of the balloon 12 in a vessel.

A stiffening member 16 extends through the shaft body 5 from at least the proximal end 7 of the shaft section 2 and has a distal end portion 17 in the balloon section 3. Preferably, the stiffening member 16 is fixed relative to the shaft body 5 against movement in its longitudinal direction, for instance at the proximal end of the shaft section 2 or in the handle 9. Instead or in addition, the stiffening member 16 may also be fixed to the handle 9, to a stiffening tube 18 projecting from the handle 9 or be fixed to the shaft body 5 at one or more other locations.

A guidewire lumen 19 extends from a proximal guidewire port 20 at a proximal end of the balloon 12, through the balloon carrier 11 and the balloon 12, to a distal guidewire port 21 near a distal end of the balloon 12. The distal guidewire port 21 is located distally of the balloon 12, so that a catheter portion without balloon projects distally of the balloon 12 to facilitate advancing of the catheter through narrow or tortuous vessels. A wall 22 between the guidewire lumen19 and the inflation lumen 6 has a portion 23 oriented obliquely away from the proximal guidewire port 20 in a section of the catheter between proximal and distal ends 24, 25 of the proximal guidewire port 20. The distal end portion 17 of the stiffening member 16 is fixed to the oblique wall portion 23. This allows the distal end portion 17 to be fixed in a simple manner to a wall portion deflecting relative to a central axis 27 of the inflation lumen 6. The stiffening effect of the stiffening member 16 is directly transferred from the stiffening member 16 to the oblique wall portion 23 of the balloon carrier 11, and accordingly effectively transferred to a guidewire 26 extending in the guidewire lumen 19 bounded by the balloon carrier 11. The section of the guidewire 26 distally of the stiffening member 16 is generally in alignment with the stiffening member 16. Therefore, pushing forces exerted by the stiffening member 16 are effectively transferred via the oblique wall portion 23 and further via the wall 22, that is generally located distally in line with the stiffening member axis, to the balloon carrier 11 and, accordingly, to the distal side of the balloon 12. Because the stiffening member 16 extends in the inflation lumen 6 and ends where the guidewire 26 enters, the catheter 1 can be of a very small cross section, also at and adjacent the guidewire entry port 20.

Distal and proximal end portions 28, 29 of the balloon 12 are sealingly coupled to the balloon carrier 11, so both ends of the balloon are attached to the balloon carrier 11. Thus, pushing forces exerted onto the balloon carrier 11 by the stiffening member 6 fixed to the balloon carrier 11 are directly transferred through the balloon, so a flexible carrier inside the balloon 12 of a small cross-section is sufficient for transferring pushing forces while avoiding buckling and ensuring that the axial distance between the distal and proximal ends of the balloon 12 is maintained for avoiding wrinkling of the balloon 12.

It is observed that these advantages of the distal and proximal end portions of the balloon being sealingly coupled to the balloon carrier may also be achieved without the feature or features that the stiffening member extends in the inflation lumen and/or a wall between the guidewire lumen, the inflation lumen having a portion oriented obliquely away from the proximal guidewire port in a section of the catheter between proximal and distal ends of the proximal guidewire port, and/or if the distal end portion of the stiffening member is fixed to another portion of the balloon carrier than the wall portion oriented obliquely away from the proximal guidewire port. However, in combination with the stiffening member extending in the inflation lumen, a wall between the guidewire lumen and the inflation lumen having a portion oriented obliquely away from the proximal guidewire port in a section of the catheter between proximal and distal ends of the proximal guidewire port, and if the distal end portion of the stiffening member is fixed to the wall portion oriented obliquely away from the proximal guidewire port, a particularly slender construction that is easy to manufacture is obtained.

A balloon section assembly 11, 12 including the balloon carrier 11 and the balloon 12 is connected to the shaft body 5 and the proximal guidewire port 20 is formed in a portion of the balloon carrier 11. Thus, the catheter 1 can be assembled in a simple manner, without making a coupling through which the guidewire lumen 19 extends, by connecting the balloon and shaft subassemblies. In particular, this allows combining different versions of shaft and balloon assemblies for providing catheters for a wide range of applications from a relatively limited variety of subassemblies.

The shaft body 5 is attached to the balloon carrier 11 at the oblique wall portion 23, so that the oblique wall portion is partially formed by a portion of the shaft body 5 and partially formed by a portion of the balloon carrier. The balloon carrier 11 is attached to an outwardly facing surface portion of an inwardly bent portion of the shaft body 5, which allows the fixation to be made in a simple manner, preferably by welding. Shaping of the proximal end portion of the shaft body 5 to obtain an oblique end portion matching the oblique portion of the balloon carrier 11 to which the distal end portion is to be welded can be achieved by polymer melt processing as the shaft body 5 is extruded or by shaping after the shaft body 5 has been extruded, e.g. stretching with local heating.

Mutual alignment of the balloon carrier 11 and the more proximal portions of the shaft body 5 is ensured because the stiffening member 16 extends across the connection between the shaft body 5 and the balloon carrier 11. This allows the connection between the shaft body 5 and the balloon carrier 11 to be relatively flexible, which ensures that this connection is not subjected to high stresses, which is in turn advantageous for reliability of this connection. The flexibility of the connection is enhanced, because the balloon carrier 11 is connected to a deflected portion of the circumference of a distal end portion of the shaft body 5 only.

The balloon carrier 11 is integrally formed, which further contributes to ease of manufacturing, smooth and direct transmission of forces and an even distribution of flexibility over the length of the balloon carrier 11.

The distal end portion 17 of the stiffening member 16 is embedded in the oblique wall portion 23 that is oriented obliquely away from the proximal guidewire port 20. This allows the distal end portion 17 to be fixed in a particularly simple and reliable manner, preferably in an orientation in-line with a proximally adjacent portion of the stiffening member 16. To this end, a bore coaxial with the shaft body 5 may be provided in the oblique wall portion 23, into which bore the distal end portion 17 of the stiffening member 16 is inserted. Also, the distal end portion 17 of the stiffening member 16 is thus outside of the inflation lumen 6, which allows a section of the inflation lumen to the side of the distal end portion 17 (which is deflected from the central axis 27 of the catheter and relative to a proximally adjacent portion of the inflation lumen 6) to be of a smaller cross-section than would be required if that section would also have to accommodate the distal end portion of the stiffening member.

The wall portion 23 oriented obliquely away from the proximal guidewire port 20 has a thickness larger than the thickness of distally adjacent portions of the wall 22. Thus, the oblique wall portion 23 is relatively stiff, which is advantageous for transferring forces from the stiffening member 16 to the balloon carrier 11.

The inflation lumen 6 narrows in distal direction from a section proximally of the distal end portion 17 of the stiffening member 16, and more in particular, from the opening where the stiffening member enters the oblique wall portion 23. This allows keeping the catheter cross section small also in a section where the guidewire lumen 19 and the inflation lumen 6 extend next to each other. Distally of the stiffening member 16, the inflation lumen 6 does not accommodate the stiffening member 16, so in that section its cross-section can be smaller than in sections that also accommodate the stiffening member 16, while leaving a similar cross-sectional area free for the inflation medium to pass as in the more proximal sections.

The distal end of the stiffening member 16 is located between the proximal and distal ends 24 and 25 of the proximal guidewire port 20. Thus, the stiffening member ends where the guidewire 26 enters, so a gradual transition of the stiffening effect of the stiffening member 16 to the stiffening effect of the guidewire 26 is achieved, without a section with a much higher or lower stiffness in-between.

Seen in a cross-sectional plane perpendicular to a longitudinal axis 27 of the catheter 1, the distal end of the stiffening member 16 is located centrally in the catheter 1. Thus pushing forces transferred via the stiffening member 16 are exerted centrally in the catheter 1 and bending of the catheter 1 as would be induced by an off-axis pushing force vector is avoided.

The distal end portion of the stiffening member 16 is in-line with the guidewire lumen 19 portion that is spaced from the proximal guidewire port 20. By this the pushing force exerted by the stiffening member is substantially in line with the guidewire 26 and in line with the balloon carrier 11, so that also the portion of the catheter 1 distally beyond the stiffening member is pushed substantially straight forward.

The stiffening member 16 is a wire of a material having a larger specific stiffness than material of which the shaft body 5 is made, so that it contributes to the stiffness of the catheter 1, while occupying little space, yet allowing elastic bending to a relatively small radius. The wire material of the stiffening member also has a larger specific stiffness than the material of which the balloon carrier 11 is made, so that the balloon carrier 11 is held in alignment with the shaft 5, yet is flexible enough to bend for allowing it to pass through curved vessel sections.

At least at a side of the catheter 1 opposite (in circumferential sense) of the proximal guidewire port 20, a proximal end of the balloon is located proximally of the distal end 25 of the proximal guidewire port 20. Thus, the proximal end of the balloon 12 does at least extend proximally until the section of the catheter where the guidewire lumen 19 begins. This allows the inflation lumen 6 to end and open into the internal space 15 of the balloon 12 in the section of the catheter where the proximal guidewire port 20 is located and where the portion of the catheter cross section occupied by the guidewire lumen 19 increases in distal direction from the proximal end 24 of the proximal guidewire port 20. In turn, this avoids the need of an inflation lumen extending into a portion of the catheter in which also the full cross section of the guidewire lumen needs to be accommodated, so the thickness of the catheter at and distally adjacent of the proximal guidewire port 20 can be relatively small. In the present example, arranging that the proximal end of the balloon 12 extends into a section of the catheter 1, where the proximal guidewire port 20 is located is achieved in an efficient manner by providing that the guidewire port 20 extends through a proximal end portion 29, such as a sleeve, of the balloon 12.

The distal end opening 27 of the inflation lumen 6 faces in a direction with a directional component radially away from the proximal guidewire port 20, so that a smooth transition into the internal space 15 of the balloon is achieved at the distal end of the inflation lumen 6. Moreover, a smooth transition in the shape of the balloon carrier is obtained, which is advantageous for at least reducing stress concentrations at the distal end of the inflation lumen 6 when the balloon carrier 11 is subjected to a bending moment.

A proximal end portion 30 of the internal space 15 inside the balloon 12 is formed by a channel bounded by a furrow 14 in the balloon carrier 11 and a portion of the proximal end portion 29 of the balloon 12. Thus, a passage from the end 27 of the inflation lumen 6 towards the central portion of the internal space 15 of the balloon 12 is obtained that occupies a particularly small portion of the cross section of the catheter 1 inside the sleeve 29 of the balloon 12.

Within the framework of the invention many other variants than the example shown and described are conceivable. For instance, the balloon carrier may be assembled from more than one part, for instance in the form of a composite construction including an elongate reinforcing member, the balloon may extend proximally beyond the guidewire port and optionally up to the shaft body and for instance have an outer surface flush with the outer surface of the shaft body.

A catheter according to the invention can be used for many types of transluminal interventions, such as percutaneous transluminal angioplasty (PTA) in which the catheter carrying the balloon is inserted through the skin and through the lumen of the vessel to the site of the narrowing. A catheter according to the invention can be used for Peripheral Angioplasty (PA) to open a blood vessel outside the coronary arteries (e.g. an artery of the abdomen or of a leg, a renal artery or a vein) as well as for percutaneous transluminal coronary angioplasty (PTCA) to enlarge the lumen of a sclerotic coronary artery, for instance as an alternative to cardiac bypass surgery for selected patients with ischemic heart disease.

The balloon section or balloon carrier preferably has a length in a range from 1 to 20 cm (for PTCA preferably in a range from 1 to 6 cm. The external diameter of the shaft body is preferably in a range from 0.5 to 1 mm and (for PTCA preferably in a range from 0.58 to 0.8 mm). The thickness of the oblique wall portion is preferably in a range from 0.04 up to 1 mm (more preferably up to 0.5 mm) and (for PTCA preferably in a range from 0.04 to 0.5 mm (more preferably up to 0.25 mm)). In relation to the external diameter of the shaft body, the thickness of the oblique wall portion is preferably in a range from 0.04 to 1 times (more preferably up to 0.5 times) the external diameter of the shaft body and, more preferably in a range from 0.08 to 0.6 times (more preferably up to 0.3 times) the external diameter of the shaft body.

For PTCA interventions, generally the shaft section or the shaft body preferably has a length in a range from 80 cm (for transradial insertion) to 160 cm (for femoral insertion) and more preferably in a range from 90 cm to 150 cm. For PTCA, the external diameter of the balloon in folded condition is preferably in a range from 0.52 to 2 mm and more preferably in a range from 0.55 to 1.8 mm and the external diameter of the balloon in expanded condition is preferably in a range from 0.6 to 5.5 mm and more preferably in a range from 0.8 to 5 mm.

## Claims

1. A balloon catheter comprising:
a shaft body (5) bounding a shaft portion (13) of an inflation lumen (6) extending from a proximal end of the shaft body (5) to a distal end of the shaft body (5);
a balloon carrier (11) connected to and projecting distally from a distal end portion (17) of the shaft body (5) and a balloon (12) sealingly coupled to the balloon carrier (11), wherein the inflation lumen (6) communicates with an internal space (15) of the balloon (12), the balloon (12) being inflatable from a slender configuration extending tightly around the balloon carrier (11) to a radially inflated configuration; and
a stiffening member (16) extending in the inflation lumen (6) from at least the proximal end of the shaft body (5) and having a distal end portion (17);
wherein a guidewire lumen (19) extends from a proximal guidewire port (20) at or near a proximal end of the balloon (12), through the balloon carrier (11) and the balloon (12), to a distal guidewire port (21) at or near a distal end of the balloon (12);
wherein a wall (22) between the guidewire lumen (19) and the inflation lumen (6) has a portion (23) oriented obliquely away from the proximal guidewire port (20) in a section of the catheter between proximal and distal ends (24, 25) of the proximal guidewire port (20); and
wherein the distal end portion (17) of the stiffening member (16) is fixed to said wall portion (23) oriented obliquely away from the proximal guidewire port (20).

2. A balloon catheter according to claim 1, wherein distal and proximal end portions (28, 29) of the balloon (12) are sealingly coupled to the balloon carrier (11).

3. A balloon catheter according to claim 1 or 2, wherein said distal end portion (17) of said stiffening member (16) is embedded in said wall portion (23) oriented obliquely away from the proximal guidewire port (20).

4. A balloon catheter according to claim 3, wherein said wall portion (23) oriented obliquely away from the proximal guidewire port (20) has a thickness larger than a thickness of distally adjacent portions of said wall (22).

5. A balloon catheter according to any of the preceding claims, wherein, from a section proximally of the distal end portion (17) of the stiffening member (16), the inflation lumen (6) narrows in distal direction.

6. A balloon catheter according to any of the preceding claims, wherein a distal end of the stiffening member (16) is located between proximal and distal ends (24, 25) of the proximal guidewire port (20).

7. A balloon catheter according to any of the preceding claims, wherein, seen in a cross-sectional plane perpendicular to a longitudinal axis of the catheter, a distal end of the stiffening member (16) is located centrally in the catheter.

8. A balloon catheter according to any of the preceding claims, wherein the distal end portion (17) of the stiffening member (16) is in-line with at least a portion of the guidewire lumen (19) spaced from the proximal guidewire port (20).

9. A balloon catheter according to any of the preceding claims, wherein the stiffening member (16) is a wire of a material having a larger specific stiffness than material of which the shaft body (5) is made.

10. A balloon catheter according to any of the preceding claims, wherein the stiffening member (16) is a wire of a material having a larger specific stiffness than material of which the balloon carrier (11) is made.

11. A balloon catheter according to any of the preceding claims, wherein at least at a side of the catheter opposite of the proximal guidewire port (20), a proximal end of the balloon (12) is located proximally of the distal end (25) of the proximal guidewire port (20).

12. A balloon catheter according to any of the preceding claims, wherein a distal end opening (27) of the inflation lumen (6) faces in a direction with a directional component radially away from the proximal guidewire port (20).

13. A balloon catheter according to any of the preceding claims, wherein a balloon section assembly (11, 12) comprising the balloon carrier (11) and the balloon (12) is connected to a shaft member (5) and wherein the proximal guidewire port (20) is formed by an opening in the balloon carrier (11).

14. A balloon catheter according to claim 13, wherein the balloon carrier (11) is attached to the shaft (5) at said wall portion (23) oriented obliquely away from the proximal guidewire port (20).

15. A balloon catheter according to any of the preceding claims, wherein a proximal end portion (30) of the internal space (15) of the balloon (12) is bounded by a furrow (14) in the balloon carrier (11) and a portion of a proximal sleeve (29) of the balloon (12) covering said furrow (14).
